# EUROPEAN PATENT SPECIFICATION

(11) **EP 3 162 356 B1**
(45) Date of publication and mention of the grant of the patent: **26.09.2018**
(21) Application number: 16196379.8
(22) Date of filing: 28.10.2016
(51) Int. Cl.: A61K 8/06, A61K 8/31, A61K 8/33, A61Q 17/04

(54) **EFFERVESCENT SUNSCREEN COMPOSITION**
SCHÄUMENDE SONNENSCHUTZZUSAMMENSETZUNG
COMPOSITION EFFERVESCENTE POUR PROTECTION CONTRE LE SOLEIL

(30) Priority: 30.10.2015 US 201562248622 P
(43) Date of publication of application: 03.05.2017
(73) Proprietor: Johnson & Johnson Consumer Inc., Skillman, NJ 08558 (US)
(72) Inventor: ABEL, Jr. Luis Carlos, 12241-070 Sao Paulo (BR); DUFORT, Marisa, Skillman, NJ New Jersey 08778 (US); LOPES, Lucas, 12241-070 Sao Paulo (BR); MIOLLO, Bruna Helena, 12241-070 Sao Paulo (BR); RIBEIRO, Maycon, 12231-901 Sao Paulo (BR); ZAHR, Alisar, Skillman, NJ New Jersey 08778 (US)
(74) Representative: Carpmaels & Ransford LLP

(56) References cited:
- EP-A1- 1 508 326
- WO-A1-2007/002047
- WO-A1-2014/052264
- WO-A1-2015/037988

## Description

The present invention relates to topically-acceptable, sunscreen compositions containing an oil-in-water emulsion comprising a UV-absorbing compound, and a combination of propellants substantially homogeneously distributed there through, which compositions provide a phase-stable, effervescent foam when dispensed from a pressurized aerosol container.

### BACKGROUND OF THE INVENTION

The prolonged exposure to UV radiation, such as from the sun, can lead to the formation of light dermatoses and erythemas, as well as increase the risk of skin cancers, such as melanoma, and accelerate skin aging, such as loss of skin elasticity and wrinkling.

Numerous sunscreen compositions are commercially available with varying ability to shield the body from ultraviolet light. Unfortunately, many commercial sunscreens are not effervescent nor having the desirable phase stability to be applied on uneven surfaces or hard to reach areas. Accordingly, sunscreen formulations that are phase-stable, flowable and capable of forming a high volume of vigorous bubbling and effervescence with relatively low energy input are desired by the consumer.

EP 1508326 A1 discloses a method for boosting the foam of a cosmetic or dematological preparation by adding one of more UV filter substances to a foamable cosmetic or dermatological preparation. WO 2007/002047 A1 and WO 2014/052264 A1 disclose a propellant-containing cosmetic composition containing at least one UV filter. WO 2015/037988 A1 disclose an aerosol container comprising a dermatological composition comprising an oil-in-water emulsion and one or more active dermatological ingredients.

### SUMMARY OF THE INVENTION

The present invention provides compositions that include a phase-stable oil-in-water emulsion comprising a discontinuous oil phase homogenously distributed within a continuous water phase, wherein said discontinuous oil phase comprises a UV-absorbing compound; and from about 20% to about 30% by weight of the composition of a combination of at least two propellants. The propellant combination comprises at least two propellants selected from the group consisting of dimethyl ether, a hydrocarbon, or mixtures thereof, and 1, 1-difluoroethane. The compositions comprise from about 8% to about 15% by weight of each propellant included in the propellant combination. The compositions provide a phase-stable, effervescent foam when discharged from a pressurized aerosol container.

### DETAILED DESCRIPTION OF THE INVENTION

The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. As used herein, unless otherwise indicated, all alkyl, alkenyl, and alkoxy groups may be straight or branched chain groups. As used herein, unless otherwise indicated, the term "molecular weight" refers to weight average molecular weight, (Mw).

Unless defined otherwise, all concentrations refer to concentrations by weight. Also, unless defined otherwise, the term "essentially free of," with respect to a class of ingredients, refers to the particular ingredient(s) being present in a concentration less than is necessary for the particularly ingredient to be effective to provide the benefit or property for which it otherwise would be used, for example, 0.75% or less by weight, such as about 0.5% or less by weight.

By "effervescent foam", it is meant a foam dispensed from a pressurized aerosol container, where the foam structure formed exhibits a decrease at least 50% of the height of the foam structure dispensed from the container at one minute after dispensing of the foam from the container. By "phase-stable, effervescent foam", it is meant an effervescent foam which does not exhibit a noticeable layer of liquid on the surface of the effervescent foam structure, or oily droplets, or lumps in the effervescent foam structure after three months, as determined by the test method described herein.

### PROPELLANTS

Compositions of the invention include a combination of at least two propellants. In certain embodiments of the invention, the propellant combination includes liquefied and/or dissolved gases immiscible with the carrier of the composition. The liquefied and/or dissolved gases can be retained essentially completely in the liquid phase of the effervescent composition with a slow boiling occurring at temperatures which are significantly higher than the boiling points of the liquefied and/or dissolved gases at the prevailing environmental pressure. Liquefied gases, such as hydrocarbons and 1, 1-difluoroethane, and dissolved gases, such as dimethyl ether, are typically used as propellants for various types of aerosol products contained in aerosol containers. When the pressure is released by means of a valve on the aerosol container, the contents of the pressurized container are discharged and any liquefied and/or dissolved gas propellants discharged from the container vaporize essentially instantaneously as the liquefied and/or dissolved gases enter the much lower pressure zone (i.e., atmospheric pressure) outside of the container.

In certain embodiments of the invention, the liquefied gas propellants are combined with dissolved gas propellants, which also tend to vaporize instantaneously upon release from the pressurized container. The mixture of the liquefied gas propellants and the dissolved gas propellants create high volume of vigorous and relatively long lasting bubbling and effervescence in the composition as discharged. The effervescent compositions are unique in that the effervescent foam formed by the compositions are flowable and easily spreadable when applied and rubbed onto the skin and impart a pleasant cooling sensation.

According to certain embodiments of the invention, the propellant combination is substantially homogeneously distributed through the effervescent composition. Examples of liquefied gas propellants include hydrocarbons, including, but not limited to, pentane, butane, isobutane and propane, and mixtures thereof, and 1, 1-difluoroethane. Examples of mixtures of hydrocarbon propellants include a mixture of 80% butane and 20% propane, based on total weight of the propellant mixture. Examples of dissolved gas propellants are ethers which include, but are not limited to, dimethyl ether. It was surprisingly discovered that, in order to provide phase-stable compositions that provide an effervescent foam when discharged from a pressurized container into atmospheric pressure, the total concentration of the combination of the propellants must be from about 20% to about 30% by weight of the effervescent composition, and the concentration of the selected propellants contained in the propellant combination, including the 80/20 weight percent mixture of butane and propane, must be from about 8% to about 15%. When either condition is not met, the resultant composition either is not phase-stable, or does not provide an effervescent foam when discharged from a pressurized container into atmospheric pressure.

### UV-ABSORBING COMPOUND

Compositions of the present invention include an ultraviolet radiation-absorbing compound, (*i*.*e*., "UV-absorbing compound"). By "UV-absorbing compound," it is meant a compound comprising one or more UV-absorbing moieties, as discussed herein below, and that absorbs radiation in some portion of the ultraviolet spectrum (290nm-400nm), such as one having an extinction coefficient of at least about 1000 mol⁻¹ cm⁻¹, for example greater than 10,000, or 100,000, or 1,000,000 mol⁻¹ cm⁻¹, for at least one wavelength within the above-defined ultraviolet spectrum.

In certain embodiments of the invention, the UV-absorbing compound may have low water solubility. For example, in certain embodiments, the UV-absorbing compound may have a water solubility that is about 3% or less by weight, such as about 1% or less by weight. By "water solubility" it is meant the maximum weight percentage of UV-absorbing compound (relative to polymer plus water) that can be placed into 100 grams deionized water and agitated so that a clear solution is obtained and remains visually homogeneous and transparent at ambient temperature for 24 hours.

The UV-absorbing compound includes one or more UV-absorbing moieties. In one particular embodiment, the first ultraviolet-absorbing moiety is a UV-A absorbing moiety. By "UV-A absorbing moiety," it is meant a moiety that confers appreciable absorbance in the UV-A portion (320nm to 400 nm) of the ultraviolet spectrum to the UV-absorbing compound. For example, when a compound that includes the UV-absorbing polymer is cast into a film, it is possible to generate a molar extinction coefficient measured for at least one wavelength in this wavelength range of at least about 1000 mol⁻¹ cm⁻¹, such as at least about 2000 mol⁻¹ cm⁻¹, such as at least about 4000 mol⁻¹ cm⁻¹. In one embodiment, the molar extinction coefficient among at least 40% of the wavelengths in this portion of the spectrum is at least about 1000 mol⁻¹ cm⁻¹.

Examples of moieties that are UV-A absorbing include tertrahydroxybenzophenones; dicarboxydihydroxybenzophenones and alkane ester or acid halide derivatives thereof; dihydroxy-, dicarboxy-, and hydroxycarboxydibenzoylmethanes and alkane ester or acid halide derivatives thereof; dihydroxy-, dicarboxy-, and hydroxycarboxystilbenes and alkane ester or acid halide derivatives thereof; bis(hydroxystyrenyl) benzenes; bis(carboxystyrenyl)benzenes and alkane ester or acid halide derivatives thereof; dihydroxy-, dicarboxy, and hydroxycarboxycarotenes and alkane ester or acid halide derivatives thereof; 2 cyano - 3,3 - diphenyl acrylic acid, 2-ethyl hexyl ester; and any suitably functionalized species capable of absorbing ultraviolet light in the 320-400 nm range.

In one embodiment, the UV-absorbing moiety is a UV-absorbing triazole and/or a UV-absorbing benzoylmethane. In a particularly notable embodiment, the UV-absorbing moiety is a UV-absorbing triazole.

By "UV-absorbing triazole" it is meant a UV-absorbing moiety containing a five-member heterocyclic ring with two carbon and three nitrogen atoms. UV-absorbing triazoles include, for example, compounds of the formula (II) or (III): 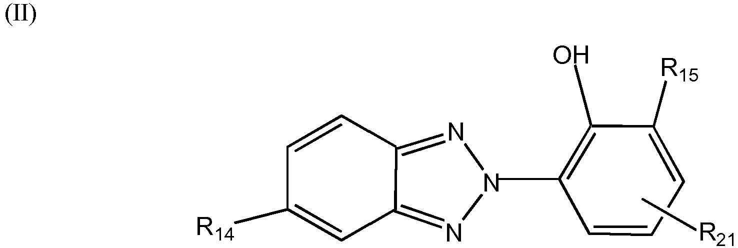 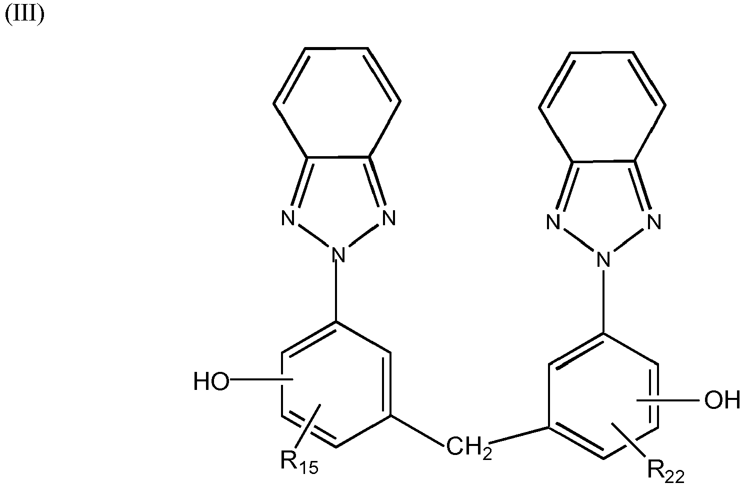 wherein R₁₄ is an optional C₁-C₁₈ alkyl or hydrogen; R₁₅ and R₂₂, independently, are optionally C₁-C₁₈ alkyl that may be substituted with a phenyl group, and R₂₁ is an optional C₁-C₈ alkyl. For (II), either of the R₁₄, R₁₅, or R₂₁ group may be oriented so as to be directly bonded to the (ester) linking group that connects the UV-absorbing dibenzoylmethane to the C-C backbone. For (III), either of the R₁₅ or R₂₂ group may be oriented so as to be directly bonded to the (ester) linking group that connects the UV-absorbing triazole to the C-C backbone.

UV-absorbing dibenzoylmethanes include those that may be represented by formula (IV): 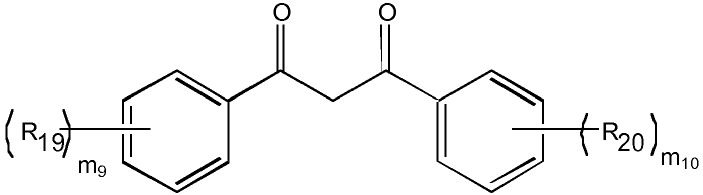 wherein R₁₉ and R₂₀, independently, are optional C₁-C₈ alkyl or C₁-C₈ alkoxy, m₉ is 0 to 3, and m₁₀ is 1 to 3. Either of the R₁₉ and R₂₀ group may be oriented so as to be directly bonded to the (ester) linking group that connects the UV-absorbing dibenzoylmethane to the C-C backbone.

Examples and the synthesis of such non-polymeric dibenzoylmethane moieties are disclosed in U.S. Patent No. 4,489,057 and include, but are not limited to, 4-(1,1-dimethylethyl)-4'-methoxydibenzoylmethane (avobenzone and sold as PARSOL 1789, Roche Vitamins and Fine Chemicals, Nutley, New Jersey, USA).

In another embodiment, the ultraviolet-absorbing moiety is a UV-B absorbing moiety. By "UV-B absorbing moiety," it is meant a moiety that confers appreciable absorbance in the UV-B portion (290nm to 320 nm) of the ultraviolet spectrum. In one embodiment, the criteria for consideration as a UV-B absorbing moiety is similar to those described above for an UV-A absorbing moiety, except that the wavelength range is 290nm to 320 nm.

Examples of suitable UV-B absorbing moieties include 4-aminobenzoic acid and alkane esters thereof; anthranilic acid and alkane esters thereof; salicylic acid and alkane esters thereof; hydroxycinnamic acid alkane esters thereof; dihydroxy-, dicarboxy-, and hydroxycarboxybenzophenones and alkane ester or acid halide derivatives thereof; dihydroxy- , dicarboxy-, and hydroxycarboxychalcones and alkane ester or acid halide derivatives thereof; dihydroxy-, dicarboxy-, and hydroxycarboxycoumarins and alkane ester or acid halide derivatives thereof; and other suitably functionalized species capable of absorbing ultraviolet light in the 290-320 nm range.

In certain embodiment of the invention, "UV-absorbing compound" may be an "organic" UV-absorber. Examples of such compounds, sometimes referred to as "monomeric, organic UV-absorbers" include, but are not limited to, methoxycinnamate derivatives such as octyl methoxycinnamate and isoamyl methoxycinnamate; camphor derivatives such as 4-methyl benzylidene camphor, camphor benzalkonium methosulfate, and terephthalylidene dicamphor sulfonic acid; salicylate derivatives such as octyl salicylate, trolamine salicylate, and homosalate; sulfonic acid derivatives such as phenylbenzimidazole sulfonic acid; benzone derivatives such as dioxybenzone, sulisobenzone, and oxybenzone; benzoic acid derivatives such as aminobenzoic acid and octyldimethyl para-amino benzoic acid; octocrylene and other β,β-diphenylacrylates; dioctyl butamido triazone; octyl triazone; butyl methoxydibenzoyl methane; drometrizole trisiloxane; menthyl anthranilate; and bis-ethylhexyloxyphenol methoxyphenyl triazine.

In yet another embodiment of the invention, UV-absorbing compounds may include UV- absorbing particles typically used at least in part to scatter ultraviolet radiation. Examples include inorganic oxides, including titanium dioxide, zinc oxide, iron oxides, silicone oxides, or other metal (e.g., transition metal, such as crystalline transition metal) oxides. Such ultraviolet screening particles are typically solid particles having a diameter from about 0.1 micron to about 10 microns.

It is further desirable that the UV-absorbing compound have an absorbance in the UV spectrum that is sufficiently high so as to make it suitable for use as a sunscreen for the human body. In one embodiment, the compound, when dissolved in a suitable solvent (e.g., DMSO, ethyl acetate, tetrahydrofuran, or the like) and spread or cast into a thin film, has a molar extinction coefficient measured for at least one wavelength within the UV spectrum, such as in the UV-A spectrum, of at least about 1000 mol⁻¹ cm⁻¹, such as at least about 2000 mol⁻¹ cm⁻¹, such as at least about 4000 mol⁻¹ cm⁻¹, or even 10,000 or 100,000 or 1,000,000 mol⁻¹ cm⁻¹.

A suitable in-vivo test method is the "Colipa Method," known to those skilled in the art. In this method, the minimum dose of solar-simulated ultraviolet radiation (UVR) required to induce a minimally perceptible erythema on human skin is determined for untreated skin and for the skin treated with the composition (erythema readings taken 24 hours after irradiation). The ratio of the dose of UV radiation needed to induce minimally perceptible erythema for the composition-protected skin (MEDp), divided by the dose required for a minimally perceptible erythema for unprotected skin (MEDu) results in the SPF value of the composition.

An irradiation apparatus used for SPF determinations is, for example, a Multiport Solar Simulator Model 601 (Solar Light Co., Philadelphia, Pennsylvania, USA) which consists of a 300 W Xenon lamp filtered with a UG11 1mm thick filter and a WG320 1mm filter (Schott Co., Philadelphia, Pennsylvania, USA) to allow exposure to UV between 240 and 800 nanometers.

### TOPICAL COMPOSITION

The effervescent compositions of the present invention may be used for a variety of cosmetic uses, especially for protection of the skin from UV radiation. The composition may be employed for various end-uses, such as recreation or daily-use sunscreens, moisturizers, cosmetics/make-up, cleansers/toners, anti-aging products, or combinations thereof. The compositions of the present invention may be prepared using methodology that is well known by an artisan of ordinary skill in the field of cosmetics formulation.

The compositions include a phase-stable oil-in-water emulsion containing a continuous water phase and a discontinuous oil phase dispersed within the continuous water phase. As used herein, "phase-stable oil-in-water emulsion" means that the water and oil phases of the oil-in-water emulsion do not noticeably separate into two phases. Phase stability of the composition containing the oil-in-water emulsion and propellant combination is determined using a glass chamber, as described herein below. In certain embodiments, the UV-absorbing compound is dissolved, as opposed to being dispersed or suspended, within the oil phase. The oil phase may be such that it is present in discrete droplets or units having an average diameter of about one micron to about 1000 microns, such as from about 1 micron to about 100 microns.

According to the present invention, the water phase may contain from about 0.75% to about 6% by weight of superhydrophilic amphiphilic copolymers and from about 1.0% to about 5% by weight of the non-UV-absorbing light-scattering particles (in suspension form), based on total weight of the composition.

The percentage by weight of water phase included in the compositions may range from about 45% to about 90%, such as from about 55% to about 80%, such as from about 60% to about 80%. The percentage by weight of water in the water phase may be about 90% or more, such as about 95% or more. In certain embodiments the percentage by weight of oil phase in the composition is from about 10% to about 55%, such as from about 20% to about 45%, such as from about 20% to about 40%

According to certain embodiments of the present invention, the oil phase consists essentially of a UV absorbing compound and the oil phase is substantially free of oil soluble polymers. By "substantially free of an oil soluble polymer", it is meant that the oil phase does not contain an oil soluble polymer at levels that will detrimentally affect the quality of foam generated by the foaming composition, or prevent the foaming composition from forming such foam when applied; for example, about 3% by weight or less, or about 1% by weight or less, or about 0.75% by weight or less. By "Oil soluble polymer" it is meant any polymeric material that is soluble in the oil phase of the composition. Examples of oil soluble polymers include, but are not limited to, a polyamide, e.g. Sylvaclear WF1500V (Polyamide-4), available from Arizona Chemical, an emollient, e.g. siliconyl carnauba wax, commercially available from Koster Keunen Inc., or oil, e.g., a film-forming polymer such as GANEX V216, commercially available from Ashland Specialty Ingredients of Wayne, NJ, and a non-ionic emulsifer such as Myrj-S100, available from Croda Inc. Silicone-based film forming polymers also may be used. In certain embodiment, the oil phase consists essentially of the UV absorbing compound. In certain embodiments, the oil phase contains about 80% or more by weight of the UV absorbing compound, such as greater than 90% by weight of the UV absorbing compound, such as greater than 95% by weight of the UV absorbing compound, such as greater than 97% by weight of the UV absorbing compound, such as greater than 99% by weight of the UV absorbing compound.

In contrast, water soluble or water dispersible polymers may be added to the compositions. The water dispersible polymers are comprised of a water-insoluble polymer that is typically micronized and dispersed into a water carrier, possibly with the use of a surface active dispersing aid. The water dispersible polymeric film formers are capable of forming a film and improving water resistance of the compositions. Examples of water soluble polymers include Polyaldo™ 10-1-L (Polyglyceryl-10 Laurate), available from Lonza. Examples of water dispersible polymeric film formers include water dispersible polyurethanes, such as Baycusan® C1000 (Polyurethane-34), available from Bayer, Dow Corning® 2501 (Bis-PEG-18 Methyl Ether Dimethyl Silane), available from Dow Corning, and Eastman AQ™ 38S (Polyester-5), available from Eastman Chemical.

In one embodiment, a composition suitable for topical/cosmetic use for application to the human body (e.g., keratinaceous surfaces such as the skin or hair), especially the skin, is provided. The composition includes one or more UV-absorbing compound described herein. In certain embodiments the concentration by weight of UV-absorbing compound in the composition is about 10% to about 50% by weight, such as from about 15% to about 40% by weight, such as from about 15% to about 35% by weight, such as from about 20% to about 30% by weight.

In certain embodiments the composition may include one or more compounds suitable for enhancing photostability. Photostabilizers include, for example, diester or polyesters of a naphthalene dicarboxylic acid.

### CARRIER

The composition may be combined with a "cosmetically-acceptable topical carrier," i.e., a carrier for topical use that is capable of having the other ingredients dispersed or dissolved therein, and possessing acceptable properties rendering it safe to use topically. As such, the composition may further include any of various functional ingredients known in the field of cosmetic chemistry, for example, emollients, as well as other ingredients commonly used in personal care compositions such as humectants, e.g., glycerin, thickeners, opacifiers, fragrances, dyes, solvents for the UV-absorbing compound, among other functional ingredients. In order to provide pleasant aesthetics, in certain embodiments of the invention, the composition is substantially free of volatile solvents, and, in particular C₁-C₄ alcohols such as ethanol and isopropanol.

Furthermore, the composition may be essentially free of ingredients that would render the composition unsuitable for topical use. As such, the composition may be essentially free of solvents such as volatile solvents, and, in particular, free of volatile organic solvents such as ketones, xylene, toluene, and the like. Further the composition may be essentially free of, or free of, propellant gas, such as hydrocarbon gases, including dimethyl ether.

### OIL-IN-WATER EMULSIFIER

Compositions of the present invention include one or more oil-in-water (O/W) emulsifiers selected from a group consisting of anionic emulsifiers and non-ionic emulsifiers. By "emulsifier," it is meant any of a variety of molecules that are suitable for emulsifying discrete oil-phase droplets in a continuous water phase. By "low molecular weight emulsifiers," it is meant emulsifiers having a molecular weight of about 2000 daltons or less, such as about 1000 daltons or less. The O/W emulsifier may be capable of lowering the surface tension of pure deionized water to 45 dynes per centimeter when added to pure deionized water at a concentration of O/W emulsifier of 0.5% or less by weight at room temperature. The O/W emulsifier may have a hydrophile-lipophile balance (HLB) that is about 8 or more, such as about 10 or more. Alternatively, the O/W emulsifier may be HLB independent. Such O/W emulsifiers contain thickeners. Examples include but not limited to Steareth-100; Steareth-2; mannan; xanthan gum.

Furthermore, the compositions of the present invention are essentially free of, or free of, monomeric surfactant. By "monomeric surfactants" it is meant any surface active agent that is monomeric. The monomeric surfactants may be anionic, nonionic, amphoteric or cationic.

In certain embodiments, the composition may include less than 10% by weight, or less than 2% by weight, of an emollient used for the prevention or relief of dryness and for the protection of the skin, as well as solubilizing the UV-absorbing compound. Suitable emollients include mineral oils, petrolatum, vegetable oils (e.g. triglycerides such as caprylic/capric triglyceride), waxes and other mixtures of fatty esters, including but not limited to esters of glycerol (e.g, isopropyl palmitate, isopropylmyristate), and silicone oils such as dimethicone. In certain embodiments, mixtures of triglycerides (e.g. caprylic/capric triclycerides) and esters of glycols (e.g. isopropyl myristate) may be used to solubilize the UV-absorbing compounds. In other embodiments of the invention, the compositions are essentially free of, or free of, emollients.

In certain embodiments, the composition includes a pigment suitable for providing color or hiding power. The pigment may be one suitable for use in a color cosmetic product, including compositions for application to the hair, nails and/or skin, especially the face. Color cosmetic compositions include, but are not limited to, foundations, concealers, primers, blush, mascara, eyeshadow, eyeliner, lipstick, nail polish and tinted moisturizers. The pigment suitable for providing color or hiding power may be composed of iron oxides, including red and yellow iron oxides, titanium dioxide, ultramarine and chromium or chromium hydroxide colors, and mixtures thereof. The pigment may be a lake pigment, e.g., an organic dye such as azo, indigoid, triphenylmethane, anthraquinone, and xanthine dyes that are designated as D&C and FD&C blues, browns, greens, oranges, reds, yellows, etc., precipitated onto inert binders such as insoluble salts. Examples of lake pigments include Red #6, Red #7, Yellow #5 and Blue #1. The pigment may be an interference pigment. Examples of interference pigments include those containing mica substrates, bismuth oxycloride substrates, and silica substrates, for instance mica/bismuth oxychloride/iron oxide pigments commercially available as CHROMALITE pigments (BASF), titanium dioxide and/or iron oxides coated onto mica such as commercially available FLAMENCO pigments (BASF), mica/titanium dioxide/iron oxide pigments including commercially available KTZ pigments (Kobo products), CELLINI pearl pigments (BASF), and borosilicate-containing pigments such as REFLECKS pigments (BASF).

The compositions of the present invention may further comprise one or more other cosmetically active agent(s). A "cosmetically active agent" is a compound that has a cosmetic or therapeutic effect on the skin, e.g., agents to treat wrinkles, acne, or to lighten the skin. The cosmetically active agent will typically be present in the composition of the invention in an amount of from about 0.001% to about 20% by weight of the composition, e.g., about 0.01% to about 10% by weight, such as about 0.1% to about 5% by weight of the composition.

The compositions of the present invention can be used by topically administering the effervescent foam to a mammal, e.g., by the direct laying on, wiping or spreading of the effervescent foam on the skin or hair of a human.

### EXAMPLES

The following examples illustrate the preparation and efficacy of compositions of the present invention.

### Example I Measurement of Effervescence and Phase Stability

The following example illustrates the effervescence and phase stability of certain compositions of the present invention. Inventive Examples E1 through E9 as shown in Tables 1 and 2 were made by the following process:
The compositions of the present invention were prepared using mixing and blending methodology that is well known by an artisan of ordinary skill. In one embodiment of the invention, an oil phase is prepared by mixing at least the UV-absorbing compound with optional oil soluble or oil-miscible ingredients. The water phase is prepared by mixing water and optional water-soluble or water-miscible ingredients together. The oil phase and the water phase are then mixed in a manner sufficient to disperse the oil phase substantially homogeneously in the water phase, such that the water phase is continuous and the oil phase discontinuous.

Comparative Examples C1 and C7 as shown in Table 3 were prepared by the process as described above.

### Phase Stability/Effervescent Test:

Phase stability and effervescence of the effervescent foam was determined as described below.

Multiple samples of each composition to be bested were prepared by filling glass containers with each mixture of the oil-in-water emulsion comprising a UV-absorbing compound and selected propellants, at selected concentrations, to be tested. The samples in the glass containers were placed under pressure of 5-6 bars and 7-8 bars, depending on test conditions described herein below, and maintained at room temperature for 24 hours. Twenty four hours after filling, the glass containers containing each sample were then placed in a water bath at 25 ± 1°C, for 45 minutes. The samples were then removed from the water bath and a foam was discharged from each pressurized container to form a foam structure having a substantially circular base and a height from the base to the top of the foam structure of about 0.5 cm. Each foam structure was then visually observed to determine any noticeable phase separation. Noticeable phase separation is indicated by formation of a liquid layer on the surface of the foam structure, or the formation of oil droplets or visible lumps in the foam structure.

The height from the base of the foam structure to the surface of the foam structure also was measured, both at the time the foam structure was discharged from the pressurized container, and again one minute after the foam structure was discharged from the pressurized container. A decrease in the height of the foam body over time is an indication that bubbles formed in the foam body upon discharge are rapidly rupturing as the propellant is released from the foam body, indicating an effervescent foam. No change, or negligible change, in the height of the foam body is an indication that bubbles formed in the foam are not rupturing as fast as the effervescent foam, which is an indication that the foam is not effervescent.

Half of the samples under 5-6 bars pressure were placed in an environmental chamber set at 5°C, while the other half of samples under 5-6 bars pressure were placed in an environmental chamber set at 25°C. Samples under 7-8 bars pressure were placed in an environmental chamber set at 45°C. The samples were maintained at the respective temperature for three months. After three months, samples were removed from the environment chambers and maintained at room temperature for 24 hours. The samples were then placed in a water bath at 25 ± 1°C, for 45 minutes. The samples were then removed from the water bath and a foam was discharged from each pressurized container to form a foam structure as described above. Each foam structure was then visually observed to determine any noticeable phase separation and the height of the foam structure both upon discharge of the foam structure from the container and one minute after discharge of the foam structure was measured.

For comparative examples C1-C7, there was no noticeable decrease in the height of the foam body after one minute. For Inventive Examples E1-E9, there was a decrease of at least 50% of the height of the foam body, and in some instances as much as an 80% decrease in the height of the foam body, e.g., the height of the foam structure decreased from 0.5 cm to 0.1 cm after one minute.

**Table 1: Formulation of the Inventive Examples**

| **Examples** | | **E1** | **E2** | **E3** |
|---|---|---|---|---|
| **Trade name** | **Ingredient (INICI Name)** | % | % | % |
| Dimethyl Ether | Dimethyl Ether | 10.00 | 15.00 | 0 |
| Butane/Propane (80/20) | Butane/Propane | 10.00 | 10.00 | 10 |
| Isobutane | Isobutane | 0 | 0 | 0 |
| 152A | 1,1-difluoroethane | 0 | 0 | 10 |
| Concentrate | Formula Lotion | 80.00 | 75.00 | 80 |
| Purified Water | Water | 56.400 | 56.400 | 56.400 |
| Versaflex V-150-PW-MW | Steareth-100; Steareth-2; Xanthan Gum; Mannan | 1.500 | 1.500 | 1.500 |
| Disodium EDTA | Disodium EDTA | 0.200 | 0.200 | 0.200 |
| Elestab CPN | Chlorphenesin | 0.000 | 0.000 | 0.000 |
| Tristat P25/ Phenoxyethnaol P25 | Phenoxyethanol | 0.500 | 0.500 | 0.500 |
| Symsave H | Hydroxyacetophenone | 0.500 | 0.500 | 0.500 |
| Hydrolite 5 | Pentylene Glycol | 1.000 | 1.000 | 1.000 |
| Structure XL | Hydroxypropyl Startch Phosphate | 0.750 | 0.750 | 0.750 |
| Neo Heliopan 303 | Octocrylene | 8.000 | 8.000 | 8.000 |
| Finsolv TN | C₁₂₋₁₅ Alkyl Benzoate | 5.000 | 5.000 | 5.000 |
| Neo Heliopan OS | Ethylhexyl Salicylate | 4.500 | 4.500 | 4.500 |
| Neo Heliopan 357 (US Sunscreen Active) | Avobenzone | 2.700 | 2.700 | 2.700 |
| Sunspheres Powder | Styrene/Acrylates Copolymer | 2.500 | 2.500 | 2.500 |
| Neo Heliopan HMS | Homosalate | 9.000 | 9.000 | 9.000 |
| Neo Heliopan BB | Benzophenone-3 | 4.500 | 4.500 | 4.500 |
| X-22 8247-D | Dimethicone; Acrylates/ Dimethicone Copolymer | 1.200 | 1.200 | 1.200 |
| Vitamin E Acetate | Tocopheryl Acetate | 0.100 | 0.100 | 0.100 |
| SymCalriol 344028 | Decylene Glycol | 0.150 | 0.150 | 0.150 |
| Abil Wax 9801 | Cetyl Dimethicone | 0.500 | 0.500 | 0.500 |
| Fragrance | Fragrance | 0.050 | 0.050 | 0.050 |
| Silisphere 6M | Silica | 1.000 | 1.000 | 1.000 |
| **TOTAL** | | 100 | 100 | 100 |
| **Effervescence Performance** | | Yes | Yes | Yes |
| **Phase Stability** | | Yes | Yes | Yes |

As shown in Table 1, Inventive examples E1, E2 and E3 exhibit superior effervescence performance and phase stability.

**Table 2: Formulation of the Inventive Examples**

| **Examples** | | **E4** | **E5** | **E6** | **E7** | **E8** | **E9** |
|---|---|---|---|---|---|---|---|
| **Trade name** | **Ingredient (INICI Name)** | % | % | % | % | % | % |
| Dimethyl Ether | **Dimethyl Ether** | 8.000 | 12.00 | 10.00 | 15.00 | 10.00 | 15.00 |
| Butane/Propane (80/20) | **Butane/Propane** | 12.000 | 8.00 | 10.00 | 15.00 | | |
| Isobutane | **Isobutane** | | | | | 10.00 | 10.00 |
| Pemulen TR1 | Acrylates/C₁₀₋₃₀Alkyl Acrylate Crosspolymer | 0.160 | 0.160 | 0.160 | 0.140 | 0.160 | 0.15 |
| EDTA DISSODIUM | EDTA DISSODIUM | 0.160 | 0.160 | 0.160 | 0.140 | 0.160 | 0.15 |
| Chlorphenesin | ELESTAB CPN | 0.200 | 0.200 | 0.200 | 0.175 | 0.200 | 0.1875 |
| SODIUM HYDROXYDE (50%) | SODIUM HYDROXYDE | 0.024 | 0.024 | 0.024 | 0.021 | 0.024 | 0.0225 |
| Sunspheres Powder | Styrene/Acrylates Copolymer | 2.000 | 2.000 | 2.000 | 1.750 | 2.000 | 1.875 |
| Neo Heliopan 303 | Octocrylene | 7.200 | 7.200 | 7.200 | 6.300 | 7.200 | 6.75 |
| Finsolv TN | C₁₂₋₁₅ Alkyl Benzoate | 4.000 | 4.000 | 4.000 | 3.500 | 4.000 | 3.75 |
| NEO HELIOPAN OS | ETHYLHEXYL SALICYLATE | 3.600 | 3.600 | 3.600 | 3.150 | 3.600 | 3.375 |
| Parsol 1789 | Butyl Methoxydibenzoylmethane | 3.200 | 3.200 | 3.200 | 2.800 | 3.200 | 3 |
| Tinosorb S | Bis-Ethylhexyloxyphenol Methoxyphenyl Triazine | 2.000 | 2.000 | 2.000 | 1.750 | 2.000 | 1.875 |
| ISOPROPYL ISOSTEARATE | PRISORINE 2021 / CRODAMOL IPIS -LQ-(MV) | 1.600 | 1.600 | 1.600 | 1.400 | 1.600 | 1.5 |
| Butylene Glycol | Butylene Glycol | 1.600 | 1.600 | 1.600 | 1.400 | 1.600 | 1.5 |
| AMPHISOL K | Cetyl Phosphate Potassium | 1.600 | 1.600 | 1.600 | 1.400 | 1.600 | 1.5 |
| ARLACEL 987 | Cera Alba; Hydrogenated Castor Oil; Sorbitan Isostearate; Stearic Acid | 1.280 | 1.280 | 1.280 | 1.120 | 1.280 | 1.2 |
| Antaron WP-660 | Triacontanyl PVP | 1.200 | 1.200 | 1.200 | 1.050 | 1.200 | 1.125 |
| Phenoxyethanol | Phenoxy ethanol | 0.560 | 0.560 | 0.560 | 0.490 | 0.560 | 0.525 |
| TRIBEHENIN | SYNCROWAX HR-C | 0.400 | 0.400 | 0.400 | 0.350 | 0.400 | 0.375 |
| SILSOFT-034 | CAPRYLYL METHYCONE | 0.160 | 0.160 | 0.160 | 0.140 | 0.160 | 0.15 |

| **Trade name** | **Ingredient (INICI Name)** | % | % | % | % | % | % |
|---|---|---|---|---|---|---|---|
| Vitamin E Acetate | Tocopheryl Acetate | 0.080 | 0.080 | 0.080 | 0.070 | 0.080 | 0.075 |
| Tinogard TT | Pentaerythrityl Tetra-di-t-butyl Hydroxyhydrocinnamate | 0.040 | 0.040 | 0.040 | 0.035 | 0.040 | 0.0375 |
| Frecolat ML | Menthyl Lactate | 0.008 | 0.008 | 0.008 | 0.007 | 0.008 | 0.0075 |
| Fragrance Zen 486213 | Parfum | 0.280 | 0.280 | 0.280 | 0.245 | 0.280 | 0.2625 |
| Benzyl alcohol | Benzyl Alcohol | 0.640 | 0.640 | 0.640 | 0.560 | 0.640 | 0.6 |
| SEPITONIC M3 | Magnesium Aspartate / Zinc Gluconate / Copper Gluconate | 0.008 | 0.008 | 0.008 | 0.007 | 0.008 | 0.0075 |
| Dow Corning CF-4444 Fluid | Dimethicone / Trisiloxane | 2.400 | 2.400 | 2.400 | 2.100 | 2.400 | 2.25 |
| Extrapone® Green Tea/Propolis 400140 | Water; Camellia Sinensis Leaf Extract; Propolis Extract; Propylene Glycol | 0.008 | 0.008 | 0.008 | 0.007 | 0.008 | 0.0075 |
| DRY FLO PURE | Aluminum Starch Octenylsuccinate | 0.400 | 0.400 | 0.400 | 0.350 | 0.400 | 0.375 |
| Purified Water | Aqua | 45.192 | 45.192 | 45.192 | 39.543 | 45.192 | 42.3675 |
| **TOTAL** | | 100 | 100 | 100 | 100 | 100 | 100 |
| **Effervescence Performance** | | Yes | Yes | Yes | Yes | Yes | Yes |
| **Phase Stability** | | Yes | Yes | Yes | Yes | Yes | Yes |

As shown in Table 2, Inventive examples E4 to E9 exhibit superior effervescence performance and phase stability.

**Table 3: Formulation of the Comparative Examples**

| **Comparative** | | **C1** | **C2** | **C3** | **C4** | **C5** | **C6** | **C7** |
|---|---|---|---|---|---|---|---|---|
| **Trade name** | **Ingredient (INCI Name)** | % | % | % | % | % | % | % |
| Dimethyl Ether | **Dimethyl Ether** | 10.00 | | | | 30.00 | | 10.00 |
| Butane/Propane (80/20) | **Butane/Propane** | | | | | | 30.00 | 20.00 |
| Isobutane | **Isobutane** | | 10.00 | 30.00 | 8.00 | | | |
| Pemulen TR1 | Acrylates/C₁₀₋₃₀ Alkyl Acrylate Crosspolymer | 0.180 | 0.180 | 0.140 | 0.184 | 0.140 | 0.140 | 0.140 |
| EDTA DISSODIUM | EDTA DISSODIUM | 0.180 | 0.180 | 0.140 | 0.184 | 0.140 | 0.140 | 0.140 |
| Chlorphenesin | ELESTAB CPN | 0.225 | 0.225 | 0.175 | 0.230 | 0.175 | 0.175 | 0.175 |
| SODIUM HYDROXYDE (50%) | SODIUM HYDROXYDE | 0.027 | 0.027 | 0.021 | 0.028 | 0.021 | 0.021 | 0.021 |
| Sunspheres Powder | Styrene/Acrylates Copolymer | 2.250 | 2.250 | 1.750 | 2.300 | 1.750 | 1.750 | 1.750 |
| Neo Heliopan 303 | Octocrylene | 8.100 | 8.100 | 6.300 | 8.280 | 6.300 | 6.300 | 6.300 |
| Finsolv TN | C₁₂₋₁₅ Alkyl Benzoate | 4.500 | 4.500 | 3.500 | 4.600 | 3.500 | 3.500 | 3.500 |
| NEO HELIOPAN OS | ETHYLHEXYL SALICYLATE | 4.050 | 4.050 | 3.150 | 4.140 | 3.150 | 3.150 | 3.150 |
| Parsol 1789 | Butyl Methoxydibenzoylmethane | 3.600 | 3.600 | 2.800 | 3.680 | 2.800 | 2.800 | 2.800 |
| Tinosorb S | Bis- Ethylhexyloxyphenol Methoxyphenyl Triazine | 2.250 | 2.250 | 1.750 | 2.300 | 1.750 | 1.750 | 1.750 |
| ISOPROPYL ISOSTEARATE | PRISORINE 2021 / CRODAMOL IPIS -LQ-(MV) | 1.800 | 1.800 | 1.400 | 1.840 | 1.400 | 1.400 | 1.400 |
| Butylene Glycol | Butylene Glycol | 1.800 | 1.800 | 1.400 | 1.840 | 1.400 | 1.400 | 1.400 |
| AMPHISOL K | Cetyl Phosphate Potassium | 1.800 | 1.800 | 1.400 | 1.840 | 1.400 | 1.400 | 1.400 |
| ARLACEL 987 | Cera Alba; Hydrogenated Castor Oil; Sorbitan Isostearate; Stearic Acid | 1.440 | 1.440 | 1.120 | 1.472 | 1.120 | 1.120 | 1.120 |
| Antaron WP-660 | Triacontanyl PVP | 1.350 | 1.350 | 1.050 | 1.380 | 1.050 | 1.050 | 1.050 |
| Phenoxyethanol | Phenoxy ethanol | 0.630 | 0.630 | 0.490 | 0.644 | 0.490 | 0.490 | 0.490 |
| TRIBEHENIN | SYNCROWAX HR-C | 0.450 | 0.450 | 0.350 | 0.460 | 0.350 | 0.350 | 0.350 |
| SILSOFT -034 | CAPRYLYL METHYCONE | 0.180 | 0.180 | 0.140 | 0.184 | 0.140 | 0.140 | 0.140 |
| Vitamin E Acetate | Tocopheryl Acetate | 0.090 | 0.090 | 0.070 | 0.092 | 0.070 | 0.070 | 0.070 |
| Tinogard TT | Pentaerythrityl Tetra-di-t-butyl Hydroxyhydrocinnam ate | 0.045 | 0.045 | 0.035 | 0.046 | 0.035 | 0.035 | 0.035 |
| Frecolat ML | Menthyl Lactate | 0.009 | 0.009 | 0.007 | 0.009 | 0.007 | 0.007 | 0.007 |
| Fragrance Zen 486213 | Parfum | 0.315 | 0.315 | 0.245 | 0.322 | 0.245 | 0.245 | 0.245 |
| Benzyl alcohol | Benzyl Alcohol | 0.720 | 0.720 | 0.560 | 0.736 | 0.560 | 0.560 | 0.560 |
| SEPITONIC M3 | Magnesium Aspartate / Zinc Gluconate / Copper Gluconate | 0.009 | 0.009 | 0.007 | 0.009 | 0.007 | 0.007 | 0.007 |
| Dow Corning CF-4444 Fluid | Dimethicone / Trisiloxane | 2.700 | 2.700 | 2.100 | 2.760 | 2.100 | 2.100 | 2.100 |
| Extrapone® Green Tea/Propolis 400140 | Water; Camellia Sinensis Leaf Extract; Propolis Extract; Propylene Glycol | 0.009 | 0.009 | 0.007 | 0.009 | 0.007 | 0.007 | 0.007 |
| DRY FLO PURE | Aluminum Starch Octenylsuccinate | 0.450 | 0.450 | 0.350 | 0.460 | 0.350 | 0.350 | 0.350 |
| Purified Water | Aqua | 50.84 1 | 50.84 1 | 39.54 3 | 51.97 1 | 39.54 3 | 39.54 3 | 39.543 |
| **DI water** | **Water** | | | | | | | |
| **TOTAL** | | 100 | 100 | 100 | 100 | 100 | 100 | 100 |
| **Effervescence Performance** | | No | No | No | No | Yes | Yes | Yes |
| **Phase Stability** | | Yes | Yes | Yes | Yes | No | No | No |

As shown in Table 3, Comparative examples (C1 to C7) exhibit either poor effervescence performance or phase stability.

**Table 4: Formulation of the Comparative Examples**

| **Comparative** | | **C8** | **C9** | **C10** |
|---|---|---|---|---|
| **Trade name** | **Ingredient (INCI Name)** | % | % | % |
| Dimethyl Ether | Dimethyl Ether | 0 | 0 | 0 |
| Butane/Propane (80/20) | Butane/Propane | 0 | 0 | 0 |
| Isobutane | Isobutane | 10.00 | 30.00 | 8.00 |
| Concentrate | Formula Lotion | 90.00 | 70.00 | 92.00 |
| Purified Water | Water | 54.800 | 54.800 | 54.800 |
| Versaflex V-150-PW-MW | Steareth-100; Steareth-2; Xanthan Gum; Mannan | 1.000 | 1.000 | 1.000 |
| Disodium EDTA | Disodium EDTA | 0.200 | 0.200 | 0.200 |
| Elestab CPN | Chlorphenesin | 0.250 | 0.250 | 0.250 |
| Tristat P25/ Phenoxyethnaol P25 | Phenoxyethanol | 0.700 | 0.700 | 0.700 |
| Symsave H | Hydroxyacetophenone | 0.000 | 0.000 | 0.000 |
| Hydrolite 5 | Pentylene Glycol | 0.000 | 0.000 | 0.000 |
| Structure XL | Hydroxypropyl Startch Phosphate | 0.750 | 0.750 | 0.750 |
| Neo Heliopan 303 | Octocrylene | 8.000 | 8.000 | 8.000 |
| Finsolv TN | C₁₂₋₁₅ Alkyl Benzoate | 5.000 | 5.000 | 5.000 |
| Neo Heliopan OS | Ethylhexyl Salicylate | 4.500 | 4.500 | 4.500 |
| Neo Heliopan 357 (US Sunscreen Active) | Avobenzone | 2.700 | 2.700 | 2.700 |
| Sunspheres Powder | Styrene/Acrylates Copolymer | 5.500 | 5.500 | 5.500 |
| Neo Heliopan HMS | Homosalate | 9.000 | 9.000 | 9.000 |
| Neo Heliopan BB | Benzophenone-3 | 4.500 | 4.500 | 4.500 |
| X-22 8247-D | Dimethicone; Acrylates/ Dimethicone Copolymer | 1.200 | 1.200 | 1.200 |
| Vitamin E Acetate | Tocopheryl Acetate | 0.100 | 0.100 | 0.100 |
| SymCalriol 344028 | Decylene Glycol | 0.150 | 0.150 | 0.150 |
| Xiameter PMX-1184 Silicone Fluid | Dimethicone; Trisiloxane | 0.500 | 0.500 | 0.500 |
| Intense Heat | Fragrance | 0.150 | 0.150 | 0.150 |
| Silisphere 6M | Silica | 1.000 | 1.000 | 1.000 |
| **TOTAL** | | 100 | 100 | 100 |
| **Effervescence Performance** | | No | No | No |
| **Phase Stability** | | Yes | Yes | Yes |

As shown in Table 4, Comparative examples C8 to C10 exhibit poor effervescence performance.

A non-exhaustive list of aspects of the disclosure is set out in the following numbered clauses.
Clause 1. A composition, comprising:
   a phase-stable oil-in-water emulsion comprising a discontinuous oil phase homogenously distributed within continuous a water phase, wherein said discontinuous oil phase comprises a UV-absorbing compound; and
   from about 20% to about 30% by weight of said composition of a combination of at least two propellants, said combination comprising at least two propellants selected from the group consisting of dimethyl ether, a hydrocarbon, and 1, 1-difluoroethane,
   wherein said composition comprises from about 8% to about 15% by weight of each of said propellants comprising said combination, and wherein said composition provides a phase-stable, effervescent foam when discharged from a pressurized container.
Clause 2. The composition of clause 1 wherein said hydrocarbon is selected from the group consisting of isobutane and a mixture of 80% butane and 20% propane, based on total weight of said mixture of butane and propane.
Clause 3. The composition of clause 2 wherein said propellant combination comprises said dimethyl ether and said mixture of butane and propane.
Clause 4. The composition of clause 2 wherein said propellant combination comprises said dimethyl ether and said isobutane.
Clause 5. The composition of clause 1 wherein said propellant combination comprises said dimethyl ether and said 1,1-difluoroethane.
Clause 6. The composition of clause 2 wherein said propellant combination comprises said 1,1-difluoroethane and said mixture of butane and propane.
Clause 7. The composition of clause 1 wherein said UV-absorbing compound comprises a UV-absorbing moiety selected from the group consisting of a UV-absorbing triazole and UV-absorbing benzoylmethane.
Clause 8. The composition of clause 1 wherein said UV-absorbing compound comprises UV-absorbing particles.
Clause 9. The composition of clause 1 wherein said UV-absorbing compound is an organic UV-absorber.
Clause 10. The composition of clause 1 wherein said discontinuous oil phase is substantially free of an oil soluble polymer.
Clause 11. The composition of clause 1 comprising an emulsifier selected from the group consisting of an anionic emulsifier and a non-ionic emulsifier.
Clause 12. The composition of clause 1 wherein said composition is essentially free of monomeric surfactants.
Clause 13. The composition of clause 1 wherein said continuous water phase comprises from about 0.75% to about 6% by weight of a superhydrophilic amphiphilic copolymer and from about 1% to about 5% by weight of a non-UV-absorbing light-scattering particle, each based on total weight of the composition.

## Claims

1. A composition, comprising:
a phase-stable oil-in-water emulsion comprising a discontinuous oil phase homogenously distributed within continuous a water phase, wherein said discontinuous oil phase comprises a UV-absorbing compound; and
from about 20% to about 30% by weight of said composition of a combination of at least two propellants, said combination comprising at least two propellants selected from the group consisting of dimethyl ether, a hydrocarbon, or mixtures thereof, and 1,1-difluoroethane,
wherein said composition comprises from about 8% to about 15% by weight of each of said propellants comprising said combination, and wherein said composition provides a phase-stable, effervescent foam when discharged from a pressurized container.

2. The composition of claim 1 wherein said hydrocarbon is selected from the group consisting of isobutane and a mixture of 80% butane and 20% propane, based on total weight of said mixture of butane and propane.

3. The composition of claim 2 wherein said propellant combination comprises said dimethyl ether and said mixture of butane and propane.

4. The composition of claim 2 wherein said propellant combination comprises said dimethyl ether and said isobutane.

5. The composition of claim 1 wherein said propellant combination comprises said dimethyl ether and said 1,1-difluoroethane.

6. The composition of claim 2 wherein said propellant combination comprises said 1,1-difluoroethane and said mixture of butane and propane.

7. The composition of any of the preceding claims wherein said UV-absorbing compound comprises a UV-absorbing moiety selected from the group consisting of a UV-absorbing triazole and UV-absorbing benzoylmethane.

8. The composition of any of claims 1 to 6 wherein said UV-absorbing compound comprises UV-absorbing particles.

9. The composition of any of claims 1 to 6 wherein said UV-absorbing compound is an organic UV-absorber.

10. The composition of any of the preceding claims wherein said discontinuous oil phase is substantially free of an oil soluble polymer.

11. The composition of any of the preceding claims comprising an emulsifier selected from the group consisting of an anionic emulsifier and a non-ionic emulsifier.

12. The composition of any of the preceding claims wherein said composition is essentially free of monomeric surfactants.

13. The composition of claim 1 wherein said continuous water phase comprises from about 0.75% to about 6% by weight of a superhydrophilic amphiphilic copolymer and from about 1% to about 5% by weight of a non-UV-absorbing light-scattering particle, each based on total weight of the composition.

## Patentansprüche

1. Zusammensetzung, umfassend:
eine phasenstabile Öl-in-Wasser-Emulsion, umfassend eine diskontinuierliche Ölphase, die homogen in einer kontinuierlichen Wasserphase verteilt ist, wobei die diskontinuierliche Ölphase eine UV-absorbierende Verbindung umfasst; und
von etwa 20 Gew.-% bis etwa 30 Gew.-%, bezogen auf die Zusammensetzung, an einer Kombination von wenigstens zwei Treibmitteln, wobei die Kombination wenigstens zwei Treibmittel ausgewählt aus der Gruppe bestehend aus Dimethylether, einem Kohlenwasserstoff oder Gemischen davon, und 1,1-Difluorethan umfasst,
wobei die Zusammensetzung von etwa 8 Gew.-% bis etwa 15 Gew.-% an jedem der Treibmittel, die die Kombination bilden, umfasst und wobei die Zusammensetzung einen phasenstabilen sprudelnden Schaum liefert, wenn sie aus einem druckbeaufschlagten Behälter ausgegeben wird.

2. Zusammensetzung gemäß Anspruch 1, wobei der Kohlenwasserstoff ausgewählt ist aus der Gruppe bestehend aus Isobutan und einem Gemisch von 80 % Butan und 20 % Propan, bezogen auf das Gesamtgewicht des Gemischs von Butan und Propan.

3. Zusammensetzung gemäß Anspruch 2, wobei die Treibmittelkombination den Dimethylether und das Gemisch von Butan und Propan umfasst.

4. Zusammensetzung gemäß Anspruch 2, wobei die Treibmittelkombination den Dimethylether und das Isobutan umfasst.

5. Zusammensetzung gemäß Anspruch 1, wobei die Treibmittelkombination den Dimethylether und das 1,1-Difluorethan umfasst.

6. Zusammensetzung gemäß Anspruch 2, wobei die Treibmittelkombination das 1,1-Difluorethan und das Gemisch von Butan und Propan umfasst.

7. Zusammensetzung gemäß einem der vorstehenden Ansprüche, wobei die UV-absorbierende Verbindung eine UV-absorbierende Einheit ausgewählt aus der Gruppe bestehend aus einem UV-absorbierenden Triazol und UV-absorbierendem Benzoylmethan umfasst.

8. Zusammensetzung gemäß einem der Ansprüche 1 bis 6, wobei die UV-absorbierende Verbindung UV-absorbierende Partikel umfasst.

9. Zusammensetzung gemäß einem der Ansprüche 1 bis 6, wobei die UV-absorbierende Verbindung ein organischer UV-Absorber ist.

10. Zusammensetzung gemäß einem der vorstehenden Ansprüche, wobei die diskontinuierliche Ölphase im Wesentlichen frei von einem öllöslichen Polymer ist.

11. Zusammensetzung gemäß einem der vorstehenden Ansprüche, umfassend einen Emulgator ausgewählt aus der Gruppe bestehend aus einem anionischen Emulgator und einem nichtionischen Emulgator.

12. Zusammensetzung gemäß einem der vorstehenden Ansprüche, wobei die Zusammensetzung im Wesentlichen frei von monomeren Tensiden ist.

13. Zusammensetzung gemäß Anspruch 1, wobei die kontinuierliche Wasserphase von etwa 0,75 Gew.-% bis etwa 6 Gew.-% an einem superhydrophilen amphiphilen Copolymer und von etwa 1 Gew.-% bis etwa 5 Gew.-% an einem nicht-UV-absorbierenden lichtstreuenden Partikel umfasst, jeweils bezogen auf das Gesamtgewicht der Zusammensetzung.

## Revendications

1. Composition, comprenant :
une émulsion huile dans eau à phases stables comprenant une phase huileuse discontinue distribuée de façon homogène dans une phase aqueuse continue, dans laquelle ladite phase huileuse discontinue comprend un composé absorbant les UV ; et
d'environ 20 % à environ 30 % en poids de ladite composition d'une combinaison d'au moins deux propulseurs, ladite combinaison comprenant au moins deux propulseurs choisis dans le groupe constitué de l'éther diméthylique, d'un hydrocarbure, ou des mélanges de ceux-ci, et du 1,1-difluoroéthane,
ladite composition comprenant d'environ 8 % à environ 15 % en poids de chacun desdits propulseurs constituant ladite combinaison, et ladite composition formant une mousse effervescente à phases stables lorsqu'elle est évacuée depuis un récipient sous pression.

2. Composition selon la revendication 1, dans laquelle ledit hydrocarbure est choisi dans le groupe constitué de l'isobutane et d'un mélange de 80 % de butane et 20 % de propane, sur la base du poids total dudit mélange de butane et de propane.

3. Composition selon la revendication 2, dans laquelle ladite combinaison de propulseurs comprend ledit éther diméthylique et ledit mélange de butane et de propane.

4. Composition selon la revendication 2, dans laquelle ladite combinaison de propulseurs comprend ledit éther diméthylique et ledit isobutane.

5. Composition selon la revendication 1, dans laquelle ladite combinaison de propulseurs comprend ledit éther diméthylique et ledit 1,1-difluoroéthane.

6. Composition selon la revendication 2, dans laquelle ladite combinaison de propulseurs comprend ledit 1,1-difluoroéthane et ledit mélange de butane et propane.

7. Composition selon l'une quelconque des revendications précédentes, dans laquelle ledit composé absorbant les UV comprend un fragment absorbant les UV choisi dans le groupe constitué d'un triazole absorbant les UV et de benzoylméthane absorbant les UV.

8. Composition selon l'une quelconque des revendications 1 à 6, dans laquelle ledit composé absorbant les UV comprend des particules absorbant les UV.

9. Composition selon l'une quelconque des revendications 1 à 6, dans laquelle ledit composé absorbant les UV est un absorbeur d'UV organique.

10. Composition selon l'une quelconque des revendications précédentes, dans laquelle ladite phase huileuse discontinue est sensiblement exempte d'un polymère soluble dans l'huile.

11. Composition selon l'une quelconque des revendications précédentes comprenant un émulsifiant choisi dans le groupe constitué d'un émulsifiant anionique et d'un émulsifiant non ionique.

12. Composition selon l'une quelconque des revendications précédentes, ladite composition étant pratiquement exempte de tensioactifs monomères.

13. Composition selon la revendication 1, dans laquelle ladite phase aqueuse continue comprend d'environ 0,75 % à environ 6 % en poids d'un copolymère amphiphile superhydrophile et d'environ 1 % à environ 5 % en poids d'une particule diffusant la lumière et n'absorbant pas les UV, chacun sur la base du poids total de la composition.
